Europäisches Patentamt
European Patent Office
Office européen des brevets

(19)

(11) Publication number: 0 460 690 A2

(12)

# EUROPEAN PATENT APPLICATION

(21) Application number: 91109337.5

(22) Date of filing: 07.06.91

(51) Int. Cl.5: A61K 31/255

(30) Priority: 07.06.90 JP 150439/90

(43) Date of publication of application:
11.12.91 Bulletin 91/50

(84) Designated Contracting States:
BE CH DE DK ES FR GB IT LI NL SE

(71) Applicant: THE GREEN CROSS CORPORATION
3-3, Imabashi 1-chome Chuo-ku
Osaka-shi Osaka(JP)

(72) Inventor: Matsuoka, Yasushi, c/o The Green Cross Corp.
Central Research Lab. 1180-1, Shodaiohtani 2-chome
Hirakata-shi, Osaka(JP)
Inventor: Inoue, Yoshihisa, c/o The Green Cross Corp.
Central Research Lab. 1180-1, Shodaiohtani 2-chome
Hirakata-shi, Osaka(JP)
Inventor: Lee, Lyang-Ja, c/o The Green Cross Corp.
Central Research Lab. 1180-1, Shodaiohtani 2-chome
Hirakata-shi, Osaka(JP)
Inventor: Sugiura, Masanori, c/o The Green Cross Corp.
Central Research Lab. 1180-1, Shodaiohtani 2-chome
Hirakata-shi, Osaka(JP)
Inventor: Hirama, Minoru, c/o The Green Cross Corp.
Central Research Lab. 1180-1, Shodaiohtani 2-chome
Hirakata-shi, Osaka(JP)

(74) Representative: Hansen, Bernd, Dr. Dipl.-Chem. et al
Hoffmann, Eitle & Partner Patent- und Rechtsanwälte Arabellastrasse 4 Postfach 81 04 20
W-8000 München 81(DE)

## (54) Use of phenolsulfophthaline derivatives for the treatment of osteoporosis.

(57) A method for treating osteoporosis comprising administering a phenolsulfophthalein compound, which has an effect of increasing bone mass, which promotes the normal osteogenesis while showing relieved side effects.

EP 0 460 690 A2

## FIELD OF THE INVENTION

This invention relates to a method for treating osteoporosis comprising administering a phenolsulfophthalein compound as an active ingredient, which has an effect of increasing bone mass and a composition for treating osteoporosis compsiring said compound.

## BACKGROUND OF THE INVENTION

Osteoporosis is a recognized disease wherein unbalanced osteogenesis and bone resorption due to metabolic disorder, endocrinopathy or aging, cause a loss in the amount of bones and, as a consequence, the bones become porous and less dense. The symptoms of osteoporosis include serious lumbar/back ache and a fracture caused by the high porosity of bones. As is often the case, osteoporosis makes the aged bedridden and can even ultimately contribute to causing death.

A remedy for osteoporosis is expected to show effects of relieving lumbar/back ache, suppressing bone resorption or increasing bone mass. However, drugs employed at present (for example, calcitonin, vitamin D, calcium preparation) each merely function ro relieve lumbar/back ache or suppress bone resorption but none of them vigorously promotes osteogenesis. Osteogenesis needs to be performed in a well-balanced state together with bone resorption. Thus, there is a risk that the suppression of bone resorption might inadvertently inhibit osteogenesis. Further, the prolonged use of estrogen, which has also been recognized as a drug which promotes osteogenesis, is accompanied by serious drawbacks including causing adverse side effects on the uterus and carcinogenesis. Accordingly, an urgent demand has grown to develop a drug which can promote normal osteogenesis while showing relieved side effects to thereby cure osteoporosis in a radically new manner.

On the other hand, it has been reported that a commercially available phenolsulfophthalein (phenol red), which has been used as a pH indicator, was bound to an estrogen receptor in mastocarcinoma [Proc. Natl. Acad. Sci. USA, 83, 2496-2500 (1986)].

Further, a compound binding to an estrogen receptor was isolated and purified from a commercially available phenolsulfophthalein and it has been reported that this compound had the following phenolsulfophthalein-like structure [J. Med. Chem., 31, 1978-1983 (1988)]:

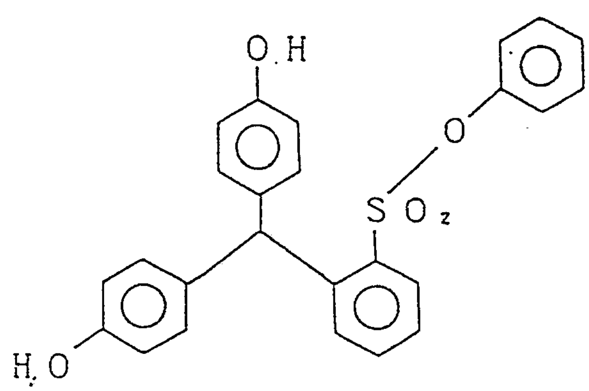

## SUMMARY OF THE INVENTION

The present inventors have conducted extensive studies on whether a phenolsulfophthalein compound binding to an estrogen receptor is potentially useful as a remedy for osteoporosis. As a result of the studies of the present inventors, it was surprisingly found that phenolsulfophthalein compounds have an effect of increasing bone mass and thus is available as an effective remedy for osteoporosis.

Accordingly, it is an object of the present invention to provide a remedy for osteoporosis which can promote normal osteogenesis while showing relieved side effects.

## DETAILED DESCRIPTION OF THE INVENTION

The phenolsulfophthalein compound to be used in the remedy for osteoporosis of the present invention is not particularly restricted, so long as it is effective in increasing bone mass. A compound having an

estrogen-like activity usually may be employed. Particularly preferable examples of said compound include those represented by formula (I):

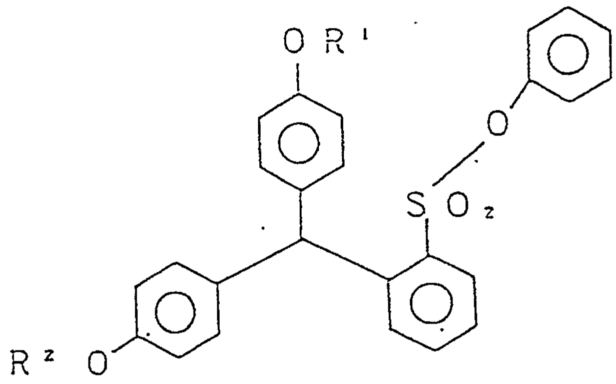

(I)

wherein $R^1$ and $R^2$ may be the same or different and each represents a hydrogen atom, an alkyl group, an acyl group or an aralkyl group.

Preferable examples of the alkyl group are those having 1 to 4 carbon atoms such as methyl, ethyl, n-propyl, i-propyl, n- butyl, i-butyl and t-butyl groups.

Preferable examples of the acyl group include aliphatic acyl groups having, in particular, 2 to 4 carbon atoms such as acetyl, propionyl and butyryl groups and aromatic acyl groups such as benzoyl and naphthoyl groups.

Preferable examples of the aralkyl group include benzyl, phenethyl and 3-phenylpropyl groups.

Among these compounds, those wherein $R^1$ and $R^2$ are the same and both represent hydrogen atoms or, alternatively, both represent acetyl groups, are particularly preferable.

The phenolsulfophthalein compound (I) may be produced by, for example, methods described in J. Med. Chem., 31, 1978-1983 (1988) or by conventional alkylation or acylation of compounds synthesized by the methods described in the aforesaid J. Med. Chem. article.

The phenolsulfophthalein compound of the present invention having an effect of increasing bone mass may be formulated into a preparation, either alone or together with known carrier(s), by a conventional method and then orally or parenterally (injection, rectoclysis or skin administration) administered.

Examples of the preparation include tablet, capsule, powder, syrup, suppository, injection and patch. The dose of the phenolsulfophthalein compound of the present invention having an effect of increasing bone mass may vary depending on the conditions, body weight, age and sex of the patient and the administration pathway. In general, it may be administered in a dose of from 0.001 to 500 mg/kg body weight one to three times per day.

In the present invention, the phenolsulfophthalein compound shows a remarkable effect of increasing bone mass. Therefore the remedy compound for treating osteoporosis as in the present invention is highly useful as a drug which promotes normal osteogenesis while showing relieved side effects.

To further illustrate the present invention, and not by way of limitation, the following Examples (Formulation Examples) and Test Examples are provided.

FORMULATION EXAMPLE 1 (Oral preparation)

| | | |
|---|---|---|
| (1) | compound 1 (in the general formula (I), $R^1$ and $R^2$ each represents both hydrogen atoms) | 5.0 mg |
| (2) | fine particles for direct punching, No. 209 (produced by Fuji Kagaku K.K., comprising 20 % of magnesium metasilicate aluminate, 30 % of corn starch and 50 % of lactose) | 46.6 mg |
| (3) | crystalline cellulose | 24.0 mg |
| (4) | CM-cellulose | 4.0 mg |
| (5) | magnesium stearate | 0.4 mg |

The above components were mixed together and the resulting mixture was punched out to obtain tablets each weighing 80 mg.

| FORMULATION EXAMPLE 2 (Intravenous injection) | |
|---|---|
| (1) compound 1 | 50 mg |
| (2) glucose | 100 mg |
| (3) physiological saline | 10 ml |

The above components were mixed together and the resulting mixture was filtered through a membrane filter. After sterile filtration, 1 ml portions of the filtrate were pipetted into vials under aseptic conditions. After filling nitrogen gas, the vials were sealed to thereby give an intravenous injection.

| FORMULATION EXAMPLE 3 (Capsule) | |
|---|---|
| (1) compound 1 | 50 g |
| (2) lactose | 935 g |
| (3) magnesium stearate | 15 g |

The above components were homogeneously mixed together. Then 200 mg portions of the powdery mixture thus obtained were filled in hard gelatin capsules.

TEST EXAMPLE 1

The effects of the phenolsulfophthalein compounds of the present invention of increasing bone mass were examined by using rats suffering from osteoporosis.

(Test method)

Wister female rats aged 7 to 8 months (8 rats per group) were subjected to ovariectomy and sciatic nerve excision to thereby cause osteoporosis. Starting from the next day after the operation, compound 1, as described above, and compound 2 (represented by formula (I), wherein $R^1$ and $R^2$ are both acetyl groups) each dispersed in 5 % Tween 20 were orally administered in a dose of 100 $\mu$g/kg body weight everyday. After 12 weeks, the thigh bones of the animals were collected and the volume, dry weight and

ash weight were measured. Thus the dry weight and ash weight per unit volume were determined.

Table 1 shows the results.

Table 1

| Group | Drug | Bone Mass: Dry wt./unit vol. ($mg/cm^3$) | Bone Mass: Ash wt./unit vol. ($mg/cm^3$) |
|---|---|---|---|
| control (normal rats) | carrier | 976 ± 11 | 387 ± 16 |
| osteoporosis | carrier | 821 ± 15 | 370 ± 19 |
| | compound 1 | 861 ± 12 | 385 ± 12 |
| | compound 2 | 856 ± 15 | 383 ± 14 |

compound 1: $R^1 = R^2 = H$.

compound 2: $R^1 = R^2 = COCH_3$.

The osteoporosis rats to which the phenolsulfophthalein compounds of the present invention were given showed improved resistance to loss of bone weight, compared with the osteoporosis rats to which a carrier alone was given.

TEST EXAMPLE 2 (Acute toxicity)

Compound 1 dispersed in 5 % Tween 20 was orally administered to eight Wister female rats aged 7 to 8 months. As a result, no animal died even at a dose of 1 g/kg body weight.

While the invention has been described in detail and with reference to specific examples thereof, it will be apparent to one skilled in the art that various changes and modifications can be made therein without departing from the spirit and scope thereof.

## Claims

1. The use of a phenolsulfophthalein compound for the manufacture of a medicament for the treatment of osteoporosis.

2. The use according to claim 1, wherein said phenolsulfophthalein compound is represented by formula (I):

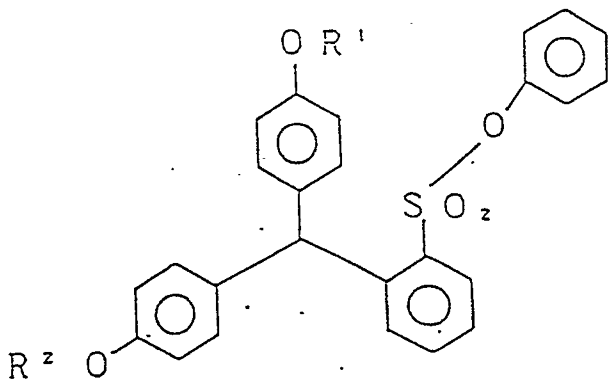

(I)

wherein $R^1$ and $R^2$ may be the same or different and each represents a hydrogen atom, an alkyl group, an acyl group or an aralkyl group.

**3.** The use according to claim 1, wherein $R^1$ and $R^2$ are the same and both represent hydrogen atoms or acetyl groups.

**4.** The use according to claim 1, wherein said phenolsulfophthalein compound is administered in a dose of from 0.001 to 500 mg/kg body weight.

**5.** A pharmaceutical composition comprising a phenolsulfophthalein compound as an active ingredient, and a pharmaceutically acceptable carrier or excipient.

**Claims for the following Contracting State: ES**

**1.** The use of a phenolsulfophthalein compound for the manufacture of a medicament for the treatment of osteoporosis.

**2.** The use according to claim 1, wherein said phenolsulfophthalein compound is represented by formula (I):

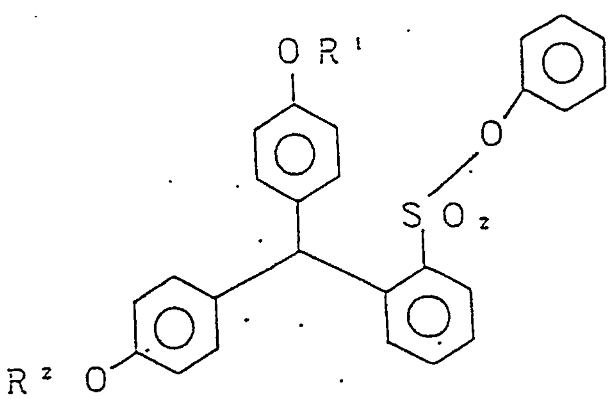

(I)

wherein $R^1$ and $R^2$ may be the same or different and each represents a hydrogen atom, an alkyl group, an acyl group or an aralkyl group.

**3.** The use according to claim 1, wherein $R^1$ and $R^2$ are the same and both represent hydrogen atoms or acetyl groups.

**4.** The use according to claim 1, wherein said phenolsulfophthalein compound is administered in a dose of from 0.001 to 500 mg/kg body weight.